# FASCICULE DE BREVET EUROPEEN

(11) **EP 2 586 468 B1**
(45) Date de publication et mention de la délivrance du brevet: **08.11.2017**
(21) Numéro de dépôt: 12290314.9
(22) Date de dépôt: 24.09.2012
(51) Int. Cl.: A61L 27/34, A61L 27/50, A61L 27/56, A61L 31/10

(54) **Implant médical poreux, par exemple bille intraculaire, et procédé de fabrication**
Poröses medizinisches Implantat, wie beispielsweise eine intraokulare Kugel, und Herstellungsverfahren
Porous medical implant, e.g. intraocular ball, and manufacturing method

(30) Priorité: 28.10.2011 FR 1103298; 16.02.2012 FR 1200448
(43) Date de publication de la demande: 01.05.2013
(73) Titulaire: POLYMEREXPERT, 33600 Pessac (FR)
(72) Inventeur: Hupin, Christophe, 33770 Salles (FR); Fouchet, Laurent, 33710 Prignac et Marcamps (FR); Dolatkhani, Marc, 33610 Cestas (FR)
(74) Mandataire: Eidelsberg, Olivier Nathan

(56) Documents cités:
- EP-A1- 0 263 012
- EP-A1- 1 374 924
- EP-A1- 1 481 694
- WO-A2-2009/150650
- US-A1- 2009 143 227
- TAPAS PAL, SHUBHAJIT PAUL, BISWANATH SA: "Polymethylmethacrylate coated alginate matrix microcapsules for controlled release of diclofenac sodium", PHARMACOLOGY & PHARMACY, vol. 2, no. 2, avril 2011 (2011-04), pages 56-66, XP002678473,

## Description

La présente invention se rapporte à un produit composite, notamment un implant, par exemple une bille intraoculaire, qui présente une bonne porosité, notamment une porosité permettant la croissance cellulaire au sein de l'implant. La présente invention se rapporte également à un procédé de fabrication d'un produit composite poreux de ce genre. La présente invention se rapporte aussi à l'utilisation d'un produit composite de ce genre dans le domaine médical.

On connaît déjà dans l'art antérieur des produits composites poreux utilisés dans le domaine médical, notamment sous la forme d'implant. Il est connu, notamment, des composites poreux en matière céramique. Ces composites poreux en matière céramique sont relativement lourds, manquent de souplesse et présentent donc des gênes pour le porteur de l'implant.

On connaît également dans l'art antérieur des produits composites poreux, qui sont réalisés à base de couches de polymères poreux qui sont fixées ensemble. Cependant, si ces produits composites présentent une plus grande souplesse que les produits composites en céramique ainsi qu'une grande porosité, ils sont cependant compliqués à fabriquer et le risque de rupture entre les entités distinctes formant le produit composite est élevé, ce qui entraîne une perte des capacités des composites.

De US 2009/143227, il est connu un implant suivant le préambule de la revendication 1.

La présente invention vise à surmonter les inconvénients de l'art antérieur en proposant un produit composite poreux simple de fabrication et qui, en outre, résiste bien à une utilisation sous fortes contraintes répétées sans entraîner de risque significatif de sa rupture, ce qui est particulièrement approprié pour une utilisation médicale en tant qu'implant.

Suivant l'invention, un implant, est tel que défini à la revendication 1 ou à la revendication 2.

Ainsi, on obtient un produit composite particulièrement résistant, le deuxième matériau ajouté au substrat de base ayant une fonction de cimentation pour maintenir la forme du substrat. On joue sur l'effet de chauffe et de refroidissement du second matériau pour obtenir le substrat dans une forme spécifique.

Suivant un mode de réalisation préféré de l'invention, la matrice est constituée en forme d'une grille.

Suivant un autre mode de réalisation préféré de l'invention, la matrice est constituée d'une feuille tissée perforée ou d'une feuille en non-tissé perforée. Suivant un mode de réalisation préféré de l'invention, le produit composite comporte une pluralité de matrices en un premier matériau polymère qui sont revêtues d'un deuxième matériau polymère et empilées les unes sur les autres.

Suivant un autre mode de réalisation préféré de l'invention, le produit composite est constitué par une matrice en le premier matériau polymère revêtue du deuxième matériau polymère et repliée sur elle-même, de préférence plusieurs fois.

De préférence, la pluralité de matrices empilées ou les plis de la matrice repliée sur elle-même sont fixés ensemble, notamment par moulage à chaud ou thermoformage.

Ainsi, suivant l'invention, on obtient des implants qui sont des produits composites qui présentent une grande porosité et qui sont faciles à fabriquer et qui, dans le même temps, résistent à des contraintes répétées sans se rompre de manière intempestive.

De préférence, la température de ramollissement du deuxième matériau polymère est supérieure à 45°C, de préférence supérieure à 50°C.

De préférence, la température de ramollissement du premier matériau polymère est supérieure à 90°C.

La présente invention se rapporte également à un procédé pour fabriquer un implant poreux, tel que défini à la revendication 11, des perfectionnement étant définis aux sous revendications 12 et 13.

Suivant un mode de réalisation préféré de l'invention, avant l'étape de thermoformage, on replie sur soi-même l'élément de base revêtu en une pluralité de plis, puis on effectue l'étape de thermoformage dans un moule ou une presse à chaud, dont la forme correspond au produit composite que l'on souhaite obtenir.

Suivant un autre mode de réalisation préféré de l'invention, on empile une pluralité de matrices de base en un premier matériau polymère revêtues du deuxième matériau polymère et on presse à chaud dans un moule ou dans une presse à chaud, dont la forme correspond à celle du produit que l'on souhaite obtenir.

Suivant l'invention, pour le second polymère, on entend aussi bien un matériau polymère que des matériaux prépolymères.

La mise en forme du produit composite est réalisée par moulage à chaud ou thermoformage à une température supérieure à la température de ramollissement (Tᵣₐₘ°C) du deuxième matériau polymère Pu ou, dans le cas de l'utilisation de prépolymères réactifs Pᵣ, à une température supérieure à leur température de réaction/réticulation (T_{rét}°C). Cette température doit, par ailleurs, être inférieure à la température de ramollissement du premier matériau polymère de la matrice (M), de façon à ce qu'elle conserve son intégrité physique, chimique et mécanique. Ces conditions impliquent que Tᵣₐₘ°C(M) > Tᵣₐₘ°C(Pᵤ) ou encore Tᵣₐₘ°C(Pᵤ) < T°C_{moulage} ou _{thermoformage}< Tᵣₐₘ°C(M) ou encore que (Tᵣₐₘ°C) (M) > T_{rét} °C (Pᵣ) ou encore T_{rét}°C (Pᵣ) < ou = T° C_{moulage} ou _{thermoformage}< Tᵣₐₘ°C(M).

Le refroidissement du moule ou de la presse à une température en dessous du point de ramollissement de Pu permet de figer et de maintenir le dispositif dans sa forme, la matrice étant emprisonnée par interpénétration du polymère (Pu) revenu à l'état vitreux ou cristallin ou du réseau tridimensionnel constitué par les prépolymères réticulés (Pᵣ). Par ailleurs, dans la mesure où il s'agit de produits composites implantables devant opérer à des températures comprises entre 35 et 42°C, une autre condition requise dans ce procédé est que le polymère (Pu) ait une température de ramollissement supérieure à la température du corps et au moins égale ou supérieure à 45°C et, de préférence, égale ou supérieure à 50°C.

Dans le cas de l'utilisation de prépolymères (Pᵣ), la matrice (M) est emprisonnée dans le réseau tridimensionnel formé par (Pᵣ).

Les matrices sont sélectionnées en fonction d'un ensemble de paramètres tels que nature chimique, biocompatibilité, biodégradabilité ou non biodégradabilité dans le corps, caractéristiques mécaniques et thermomécaniques, notamment dans son milieu d'implantation (effet du pH, de l'humidité), structure, texture, maillage, type et densité de pores ou perforation du tissus ou du non-tissé polymère. Un autre paramètre important est l'aptitude des surfaces des polymères de base formant la ou les matrices à fixer le polymère P(Pᵤ ou Pᵣ) de façon stable. Un traitement par plasma ou corona, ou tout autre traitement physique ou chimique de surface permettant d'accroître la fixation du polymère (P) peut être appliqué pour améliorer sa fixation. De préférence, la température de ramollissement de la matrice (M) doit être supérieure à 70°C et de préférence supérieure à 90°C. A titre d'exemples non limitatifs, des tissés et non-tissés à base de polypropylènes, de polyéthylènes, de polyamides, de polyesters, de polyuréthanes sont particulièrement bien adaptés pour cette fonction.

De préférence, la dimension de la section des perforations formées dans la matrice de base est comprise entre 0,2mm² et 10mm², notamment entre 4 mm² et 8 mm².

L'épaisseur du deuxième matériau formant le revêtement de la matrice de base est compris de préférence entre 500 et 1000 microns, notamment entre 650 et 750 microns, de sorte que la ou les dimensions des perforations du produit final, constitué de la matrice de base perforée et du revêtement, diffèrent très peu de celle (s) de la matrice de base.

De préférence, le polymère (Pᵤ) est choisi parmi les polymères thermoplastiques amorphes ou semi-cristallins présentant une température de ramollissement (transition vitreuse ou température de fusion supérieure à 45°C) et, de préférence, supérieure à 50°C. Sa nature chimique, sa biocompatibilité, sa biodégradabilité ou non biodégradabilité dans le corps, ses caractéristiques mécaniques et thermomécaniques, notamment dans le corps (effet du pH, de l'eau), son aptitude à adhérer sur la surface de M, non modifiée ou après traitement et à fixer le polymère (Pu) de façon stable. A titre d'exemples non limitatifs, on peut citer comme deuxième matériau polymère les polyméthacrylates les copolymères méthacrylate-acrylate, les polyéthylènes, basse et moyenne densité, les copolymères éthylène-oléfine, la polycaprolactone et ses copolymères avec les lactones, les polyéthers.

De préférence, les prépolymères réactifs (Pᵣ) sont choisis parmi les systèmes, polyol/diisocyanate, polyamine/diisocyanate, les époxys, et tout autre polymère porteur de fonctions réactives permettant la réticulation et la formation d'un réseau tridimensionnel.

Des produits composites de différentes formes peuvent être obtenus en fonction du moule utilisé. On peut ainsi réaliser des sphères poreuses utilisables comme prothèses oculaires dans les cas d'énucléation ou éviscération de l'oeil.

La présente invention vise aussi une bille intraoculaire comportant un produit composite de l'invention.

On peut également réaliser des disques poreux utilisables en chirurgie du rachis comme substitut de disque intervertébral. Ces disques peuvent avoir diverses formes permettant leur introduction par simple incision et leur positionnement aisé, mais également présenter une compressibilité ajustée, notamment par introduction au coeur du disque d'une forme métallique ou en céramique ou en polymère aux propriétés mécaniques spécifiques (forme rigide, souple, creuse, pleine).

On peut aussi utiliser la technique de la présente invention pour réaliser des prothèses utilisables en reconstruction faciale en chirurgie réparatrice ou esthétique. Ainsi, il est possible d'élaborer un moule permettant de réaliser des formes pouvant servir de prothèse du menton, de la paroi nasale, ou encore des joues. La seule limitation dans le design de ces prothèses étant l'impossibilité de réaliser par moulage ou par pressage à chaud la forme appropriée pour la prothèse de substitution. Les propriétés des prothèses peuvent, en outre, être facilement modulées au plan de la rigidité et de la tenue mécanique par le choix de la matrice et du polymère d'enduction.

La porosité de ces prothèses leur apporte une grande légèreté et permet de les fixer par suture avant que la croissance de tissus dans les pores ouverts n'assure une cohésion parfaite de la prothèse avec son environnement

La présente invention se rapporte également à l' utilisation du procédé suivant l'invention pour fabriquer un disque intervertébral ou une bille intra oculaire.

Le procédé d'élaboration des produits composites et les caractéristiques de ces produits composites sont décrits en détails ci-après par une série d'exemples de différents types d'application donnés à titre d'illustration uniquement.

### Exemples

### 1. Elaboration de billes poreuses à base d'une matrice de polypropylène (PP) enduite de polyméthacrylate de méthyle (PMMA)

Une solution de PMMA (référence Degacryl 6606F, Degussa) à 10% massique dans la méthyle éthyle cétone (MEK) est préparée dans un ballon de 2L sous agitation, puis placée dans un récipient à grande ouverture. Une bande d'une grille de polypropylène de 150 mm X 340 mm (référence PPKM802 de Textile Development Associate, TDA) maintenue aux deux extrémités est alors immergée dans le récipient contenant la solution de PMMA, puis séchée sous flux d'air pour éliminer l'excès de PMMA susceptible de boucher certains pores de la grille. Une seconde immersion de la grille dans le sens inverse du premier est ensuite réalisée, suivie d'un séchage sous courant d'air, puis la grille est séchée à 50°C. Cette procédure peut être renouvelée pour accroître la quantité de PMMA fixée sur la grille. La grille est finalement séchée dans une étuve sous vide à 80°C durant 4 h 30. Après trois immersions, le grammage de l'enduction est déterminé par pesée.

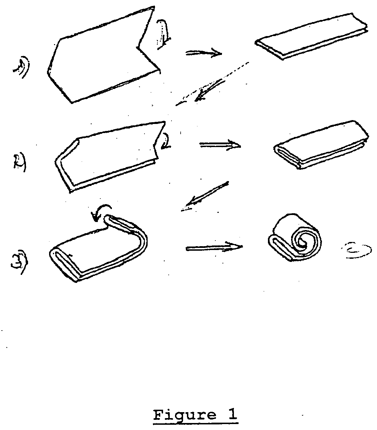

Une préforme de 120 mm X 90 mm est découpée dans la grille de PP enduite, puis celle-ci est pliée selon le protocole précis représenté à la Figure 1.

Deux pliages sont réalisés dans le sens horizontal, puis quatre, toujours dans le sens horizontal. La bande obtenue est ensuite enroulée sur elle-même, afin d'obtenir un rouleau dense de matière. Celui-ci est inséré dans la partie basse d'un moule cylindrique. Le volume de la cavité du moule permet de déterminer la taille de la bille.

La partie supérieure du moule ou piston est ensuite insérée et une rotation de 360° appliquée, puis le piston est retiré. Cette action est répétée trois fois afin de bien disposer le polymère. Le piston est finalement inséré dans le conduit du moule et le moule est placé entre les deux mâchoires d'une presse chauffante qui est portée à 130°C, c'est à dire à une température supérieure à la température de ramollissement du PMMA et inférieure à celle du polypropylène. Le moule est maintenu à chaud pendant 10 minutes puis le moule est laissé à refroidir jusqu'à 50°C, après quoi le dispositif en forme de bille peut être démoulé et récupéré.

Après démoulage, les disques sont immergés dans de l'éthanol absolu durant une heure, puis séchés sous vide durant douze heures, afin d'éliminer toutes traces de composés volatils.

La porosité de la bille est supérieure à 50%.

Les mesures de porosité par porosimètrie au mercure confirment que la grande majorité des pores est accessible depuis l'extérieur.

Ces billes trouvent des applications en tant que prothèse oculaire. La taille et la porosité des billes peuvent être déclinées dans une large gamme permettant notamment de couvrir les besoins en prothèse oculaire.

### 2. Elaboration de disques poreux compressibles à base d'une matrice de polypropylène (PP) enduite de polyméthacrylate de méthyle (PMMA) et possédant une forme à coeur

Une solution de PMMA (référence Degacryl 6606F, Degussa) à 10% massique dans la méthyle éthyle cétone (MEK) est préparée dans un ballon de 2L sous agitation, puis placée dans un récipient à grande ouverture. Une préforme est découpée dans une grille de polypropylène de 120 mm X 90 mm (référence PPKM802 de Textile Development Associate, TDA) est découpée et un préforme sphérique en PEEK de diamètre 5 mn et de hauteur 5 mn environ est placée en son centre et fixée à la grille.

L'ensemble constituée par la préforme sur laquelle est accroché le disque, est alors immergée dans le récipient contenant la solution de PMMA, puis séchée sous flux d'air pour éliminer l'excès de PMMA susceptible de boucher certains pores de la grille. Une seconde immersion du dit ensemble dans le sens inverse du premier est ensuite réalisée, suivie d'un séchage sous courant d'air, puis la grille est séchée à 50°C. Cette procédure peut être renouvelée pour accroître la quantité de PMMA fixée sur la préforme de polypropylène. L'ensemble est finalement séché dans une étuve sous vide à 80°C durant 4 h 30.

La grille de PP enduite contenant le disque est pliée selon un protocole précis, de façon à maintenir la forme centrale au coeur de la matrice de polypropylène. La bande obtenue est ensuite enroulée sur elle même afin d'obtenir un rouleau dense de matière. Celui-ci est inséré dans la partie basse d'un moule cylindrique.

La partie supérieure du moule ou piston est ensuite insérée dans le conduit du moule et le moule est placé entre les deux mâchoires d'une presse chauffante qui est portée à 130°C, c'est à dire à une température supérieure ou égale à la température de ramollissement du PMMA (Tram∼100°C) et inférieure à celle du polypropylène (Tram∼160°C). Le moule est maintenu à chaud pendant dix minutes, puis le moule est laissé à refroidir jusqu'à 50°C, après quoi le dispositif en forme de bille poreuse peut être démoulé et récupéré.

Après démoulage, les dispositifs sont immergés dans de l'éthanol absolu durant une heure, puis séchés sous vide durant douze heures afin d'éliminer toutes traces de composés volatils.

### 3. Elaboration de disques poreux compressibles à base d'une matrice de polypropylène (PP) enduite de polyuréthane (PU)

Un co-oligomère pluri-hydroxy fonctionnel à base de MMA et de 2-hydroxyéthyl méthacrylate (HEMA)(masse molaire moyenne 1500 g/mole, f_{OH} = 3) synthétisé par voie radicalaire dans nos laboratoires est solubilisé dans la méthyl éthyl cétone et placé dans un ballon de 2L sous agitation et mélangé avec de l'hexaméthylène di-isocyanate de façon à avoir une stoechiométrie entre fonctions hydroxyle et isocyanate. Une bande d'une grille de polypropylène de 120 mm X 90 mm, maintenue aux deux extrémités, est alors immergée dans le récipient contenant le mélange. Une seconde immersion de la grille dans le sens inverse du premier est ensuite réalisée suivie d'une évaporation de la MEK à 30°C sous courant d'air sec puis sous vide partiel.

Une préforme est découpée dans la grille de PP enduite puis celle-ci est pliée selon un protocole précis est insérée dans la partie basse d'un moule en forme de disque.

La partie supérieure du moule ou piston est ensuite insérée dans le conduit du moule et le moule est placé entre les deux mâchoires d'une presse chauffante qui est portée à 130°C, c'est à dire à une température supérieure à la température de ramollissement du PU et inférieure à celle du polypropylène. Le moule est maintenu à chaud pendant six heures, puis le moule est laissé à refroidir jusqu'à 50°C, après quoi le dispositif en forme de disque peut être démoulé et récupéré.

Après démoulage, les disques sont immergés dans de l'éthanol absolu durant douze heures, pour éliminer toutes traces de fonctions isocyanate n'ayant pas réagies, puis séchés sous vide durant douze heures, afin d'éliminer toutes traces de composés volatils.

### 4. Elaboration de disques poreux compressibles à base d'une matrice de polypropylène (PP) enduite d'un mélange réactif de monomères

Un mélange de monomères, méthacrylate de méthyle (80% massique), acrylate de butyle (15% massique), et diméthacrylate de méthyle (3% massique) et d'un catalyseur radicalaire le peroxyde de benzoyle (2% massique) est placé dans un ballon de 2L sous agitation. Une préforme découpée dans une grille de polypropylène de 120 mm X 90 mm est alors immergée dans le récipient contenant le mélange. Une seconde immersion de la grille dans le sens inverse du premier est ensuite réalisée. Après avoir été égouttée pour retirer l'excès des monomères, la préforme de PP enduite est insérée dans la partie basse d'un moule en forme de disque. La partie supérieure du moule est ensuite insérée et le moule est placé entre les deux mâchoires d'une presse chauffante qui est portée à 100°C, c'est à dire à une température supérieure à la température de ramollissement du mélange de monomères et inférieure à celle du polypropylène. Le moule est maintenu à chaud pendant dix heures puis le moule est laissé à refroidir jusqu'à 50°C, après quoi le dispositif en forme de disque peut être démoulé et récupéré.

Après démoulage, les disques sont immergés dans de l'éthanol absolu durant douze heures, pour éliminer toutes traces de monomères n'ayant pas réagis, puis séchés sous vide durant douze heures, afin d'éliminer toutes traces de composés volatils.

### 5. Elaboration de disques poreux compressibles à base d'une matrice de polypropylène (PP) enduite d'un mélange réactif de monomères contenant un agent de contraste

A un mélange de monomères, identique à celui de l'exemple 4 (73% massique) et d'un catalyseur radicalaire, le peroxyde de benzoyle (2% massique) placé dans un ballon de 2L sous agitation est ajouté un agent de contraste l'oxyde de zirconium (25% massique). Une préforme découpée dans une grille de polypropylène de 120 mm X 90 mm est alors immergée dans le récipient contenant le mélange. Une seconde immersion de la grille dans le sens inverse du premier est ensuite réalisée.

La grille de PP enduite est insérée dans la partie basse d'un moule. La partie supérieure du moule est ensuite insérée et le moule est placé entre les deux mâchoires d'une presse chauffante qui est portée à 100°C, c'est à dire à une température supérieure à la température de ramollissement du mélange et inférieure à celle du polypropylène. Le moule est maintenu à chaud pendant dix heures, puis le moule est laissé à refroidir jusqu'à 50°C, après quoi le dispositif en forme de disque peut être démoulé et récupéré.

Après démoulage, les disques sont immergés dans de l'éthanol absolu durant douze heures, pour éliminer toutes traces de monomères n'ayant pas réagis, puis séchés sous vide durant douze heures, afin d'éliminer toutes traces de composés volatils.

Dans les exemples 1-5 ci dessus, la dimension de la section des perforations formées dans la grille est comprise entre 0,2mm² et 10mm², notamment entre 4 mm² et 8 mm². L'épaisseur de PMMA est de 700 microns à plus ou moins 50 microns.

Dans la présente description, on ne parle que d'un premier matériau polymère et d'un second matériau polymère. Cependant, chacun d'entre eux peut être constitué d'un mélange de polymères ayant chacun des caractéristiques prévues pour respectivement le premier polymère et le second polymère. Ainsi, si le premier polymère est en fait constitué d'un mélange de N premiers matériaux polymères et le second polymère est constitué d'un mélange de P seconds matériaux polymères, les conditions se rapportant aux températures de ramollissement deviennent que la plus grande des températures de ramollissement ou de réaction/réticulation des P seconds matériaux polymères est inférieure à la plus petite des températures de ramollissement des N premiers matériaux polymères et, d'autre part, que la plus basse température de ramollissement des seconds matériaux polymères de type Pu est supérieure à 45°C et de préférence supérieures à 50°C.

Telle que définie dans la présente demande, la température de ramollissement dépend du grade et des références commerciales. On définit donc plutôt un domaine de ramollissement comme suit :
Comme premier matériau pour l'élément de base, outre le polypropylène (Tram comprise entre 145° et 175°C), on peut également utiliser le Polyéthylène haute densité (Tram comprise entre 90° et 120°C), le polyéthylène basse densité (Tram comprise entre 80° et 115°C ; les polyesters tels que le Polyéthylène téréphtalate (Tram comprise entre 175° et 210°C) ou le Polybuthylène téréphtalate (Tram comprise entre 180° et 225°C) ; ou les polyamides tels que PA 6,6 (Tram comprise entre 210° et 255°C), PA 6 (Tram comprise entre 180 et 220 °C) ou PA 11 (Tram comprise entre 155 et 185°C).

Comme second matériau (revêtement), outre le PMMA, on peut utiliser un Poly(∑- caprolactone) (Tram comprise entre 45 et 50°C) ; un copolymère éthylène acétate de vynile (EVA) (Tram comprise entre 50 et 70°C, la température de ramollissement dépendant du ratio éthylène/acétate de vinyle, par exemple pour l'EVATANE 28-800 la Tram est égale à 63°).

## Revendications

1. Implant comportant au moins un élément de base ou matrice, en un premier matériau polymère, revêtu d'un revêtement en un deuxième matériau polymère, **caractérisé en ce que** le au moins un élément de base ou matrice revêtu du revêtement comporte des ouvertures le traversant de part en part ; et la température de ramollissement du deuxième matériau polymère étant inférieure à la température de ramollissement du premier matériau polymère.

2. Implant suivant la revendication 1, **caractérisé en ce que** le deuxième matériau est à base de pré-polymères et la température de réticulation du deuxième matériau est inférieure à la température de ramollissement du premier matériau polymère.

3. Implant suivant la revendication 1 ou 2, **caractérisé en ce que** la matrice est constituée en forme d'une grille.

4. Implant suivant la revendication 1 ou 2, **caractérisé en ce que** la matrice est constituée d'une feuille tissée perforée.

5. Implant suivant la revendication 1 ou 2, **caractérisé en ce que** la matrice est constituée d'une feuille en non-tissé perforée.

6. Implant suivant l'une des revendications 1 à 5, **caractérisé en ce que** l'implant comporte une pluralité de matrices revêtues du deuxième matériau de polymère empilées les unes sur les autres.

7. Implant suivant l'une des revendications 1 à 6, **caractérisé en ce que** l'implant est constitué par une matrice revêtue du deuxième matériau polymère repliée sur elle-même, de préférence plusieurs fois.

8. Implant suivant l'une des revendications 1 à 7, **caractérisé en ce que** la pluralité de matrices empilées ou les plis de la matrice repliée sur elle-même sont fixés ensemble, notamment par moulage à chaud ou thermoformage.

9. Bille intraoculaire constituée d'un implant suivant la revendication 8.

10. Disque intervertébral constitué d'un implant suivant la revendication 8.

11. Procédé pour fabriquer un implant poreux suivant- l'une des revendications précédentes qui comporte les étapes dans lesquelles on prend au moins un élément de base ou matrice(M) perforé, comportant des ouvertures qui le traversent de part en part, par exemple sous la forme d'une grille, d'une feuille tissée perforée ou non-tissée perforée en un premier matériau polymère, on revêt l'élément de base d'un deuxième matériau polymère; puis on presse à chaud ou on thermoforme l'élément de base revêtu pour obtenir un produit composite, la mise en forme du produit composite par moulage à chaud ou thermoformage étant effectuée à une température supérieure à la température de ramollissement du deuxième matériau polymère, ou dans le cas de pré-polymères réactifs , à une température supérieure à leur température de réticulation, et à une température inférieure à la température de ramollissement du premier matériau, pour ainsi conserver son intégrité physique, chimique et mécanique.

12. Procédé suivant la revendication 11, **caractérisé en ce qu'**on empile une pluralité de matrices de base en un premier matériau polymère revêtu du deuxième matériau et on presse à chaud dans un moule ou une presse à chaud, dont la forme correspond à celle du produit que l'on souhaite obtenir.

13. Procédé suivant la revendication 11, **caractérisé en ce qu'**avant l'étape de thermoformage, on replie sur soi-même l'élément de base revêtu en une pluralité de plis, puis on effectue l'étape de thermoformage dans un moule ou une presse à chaud, dont la forme correspond au produit composite que l'on souhaite obtenir.

14. Procédé suivant l'une des revendications 11 à 13 **caractérisé en ce que** l'implant poreux est un disque intervertébral.

15. Procédé suivant l'une des revendications 11 à 13 **caractérisé en ce que** l'implant poreux est une bille intraoculaire.

## Patentansprüche

1. Implantat, umfassend mindestens ein Basis- oder Matrixelement aus einem ersten Polymermaterial, das mit einer Beschichtung aus einem zweiten Polymermaterial beschichtet ist, **dadurch gekennzeichnet, dass** das mindestens eine Basis- oder Matrixelement, das mit der Beschichtung beschichtet ist, Öffnungen umfasst, die dieses von einer Seite zur anderen durchqueren; und wobei die Erweichungstemperatur des zweiten Polymermaterials niedriger ist als die Erweichungstemperatur des ersten Polymermaterials.

2. Implantat nach Anspruch 1, **dadurch gekennzeichnet, dass** das zweite Material auf Vorpolymeren basiert, und die Vernetzungstemperatur des zweiten Materials niedriger ist als die Erweichungstemperatur des ersten Polymermaterials.

3. Implantat nach Anspruch 1 oder 2, **dadurch gekennzeichnet, dass** die Matrix in Form eines Gitters gebildet ist.

4. Implantat nach Anspruch 1 oder 2, **dadurch gekennzeichnet, dass** die Matrix aus einer perforierten gewebten Schicht besteht.

5. Implantat nach Anspruch 1 oder 2, **dadurch gekennzeichnet, dass** die Matrix aus einer perforierten nichtgewebten Schicht besteht.

6. Implantat nach einem der Ansprüche 1 bis 5, **dadurch gekennzeichnet, dass** das Implantat mehrere mit dem zweiten Polymermaterial beschichtete Matrizen umfasst, die aufeinander gestapelt sind.

7. Implantat nach einem der Ansprüche 1 bis 6, **dadurch gekennzeichnet, dass** das Implantat aus einer mit dem zweiten Polymermaterial beschichteten Matrix besteht, die, vorzugsweise mehrere Male, in sich gefaltet ist.

8. Implantat nach einem der Ansprüche 1 bis 7, **dadurch gekennzeichnet, dass** die mehreren gestapelten Matrizen oder die Falten der in sich gefalteten Matrix insbesondere durch Wärmeformen oder Thermoformen aneinander befestigt sind.

9. Intraokulare Kugel, welche aus einem Implantat nach Anspruch 8 besteht.

10. Bandscheibe, welche aus einem Implantat nach Anspruch 8 besteht.

11. Verfahren zur Herstellung eines porösen Implantats nach einem der vorhergehenden Ansprüche, welches die Schritte umfasst, in denen mindestens ein perforiertes Basis- oder Matrixelement (M), umfassend Öffnungen, die dieses von einer Seite zur anderen, beispielsweise in Form eines Gitters, einer perforierten gewebten oder perforierten nichtgewebten Schicht aus einem ersten Polymermaterial durchqueren, bereitgestellt wird, das Basiselement mit einem zweiten Polymermaterial beschichtet wird; dann das beschichtete Basiselement warmgepresst oder thermogeformt wird, um ein Verbundprodukt zu erhalten, wobei die Formgebung des Verbundprodukts durch Wärmeformen oder Thermoformen bei einer Temperatur über der Erweichungstemperatur des zweiten Polymermaterials durchgeführt wird oder im Fall von reaktiven Vorpolymeren bei einer Temperatur über ihrer Vernetzungstemperatur und bei einer Temperatur unter der Erweichungstemperatur des ersten Materials, um so seine physikalische, chemische und mechanische Integrität aufrechtzuerhalten.

12. Verfahren nach Anspruch 11, **dadurch gekennzeichnet, dass** mehrere Basismatrizen aus einem ersten Polymermaterial, das mit dem zweiten Material beschichtet ist, gestapelt werden und in einer Form oder einer Wärmepresse, deren Form jener des Produkts entspricht, das erhalten werden soll, gepresst wird.

13. Verfahren nach Anspruch 11, **dadurch gekennzeichnet, dass** vor dem Thermoformschritt das beschichtete Basiselement in sich in mehrere Falten gefaltet wird, dann der Thermoformschritt in einer Form oder einer Wärmepresse, deren Form jener des Verbundprodukts entspricht, das erhalten werden soll, durchgeführt wird.

14. Verfahren nach einem der Ansprüche 11 bis 13, **dadurch gekennzeichnet, dass** das poröse Implantat eine Bandscheibe ist.

15. Verfahren nach einem der Ansprüche 11 bis 13, **dadurch gekennzeichnet, dass** das poröse Implantat eine intraokulare Kugel ist.

## Claims

1. Implant, comprising at least one base element or matrix of a first polymer material coated with a coating of a second polymer material, **characterised in that** the at least one base element or matrix coated with the coating exhibits openings passing through it from one side to the other; and the softening temperature of the second polymer material being lower than the softening temperature of the first polymer material.

2. Implant as defined in claim 1, **characterized in that** the second material is based on prepolymers and the crosslinking temperature of the second material is lower than the softening temperature of the first polymer material.

3. Implant as defined in claim 1 or 2, **characterised in that** the matrix is constituted in the form of a mesh.

4. Implant as defined in claim 1 or 2, **characterised in that** the matrix is constituted by a perforated woven sheet.

5. Implant as defined in claim 1 or 2, **characterised in that** the matrix is constituted by a perforated nonwoven sheet.

6. Implant as defined in one of claims 1 to 5, **characterised in that** the implant comprises a plurality of matrices coated with the second polymer material stacked one on top of the other.

7. Implant as defined in one of claims 1 to 6, **characterised in that** the implant is constituted by a matrix coated with the second polymer material which is folded up, preferably several times.

8. Implant as defined in one of claims 1 to 7, **characterised in that** the plurality of stacked matrices or the folds of the folded matrix are fixed together, especially by hot moulding or thermoforming.

9. Intraocular ball constituted by an implant as defined in claim 8.

10. Intervertebral disc constituted by an implant as defined in claim 8.

11. Process for the production of a porous implant as defined in one of the preceding claims, which comprises steps in which there is taken at least one base element or perforated matrix (M), comprising openings passing through it from one side to the other, for example in the form of a mesh, of a perforated woven sheet or a perforated nonwoven sheet of a first polymer material, the base element is coated with a second polymer material; then the coated base element is subjected to hot pressing or thermoforming in order to obtain a composite product, shaping of the composite product by hot moulding or thermoforming being carried out at a temperature higher than the softening temperature of the second polymer material or, in the case of reactive prepolymers, at a temperature higher than their crosslinking temperature, and at a temperature lower than the softening temperature of the first material, in order thus to conserve its physical, chemical and mechanical integrity.

12. Process as defined in claim 11, **characterised in that** a plurality of base matrices of a first polymer material coated with the second material are stacked, and hot pressing is carried out in a mould or hot press, the shape of which corresponds to that of the product that is to be obtained.

13. Process as defined in claim 11, **characterised in that**, before the thermoforming step, the coated base element is folded up in a plurality of folds, and then the thermoforming step is carried out in a mould or hot press, the shape of which corresponds to the composite product that is to be obtained.

14. Process as defined in one of claims 11 to 13, **characterized in that** the porous implant is an intervertebral disc.

15. Process as defined in one of claims 11 to 13 **characterized in that** the porous implant is in intraocular ball.
